# EUROPEAN PATENT SPECIFICATION

(11) **EP 3 466 279 B1**
(45) Date of publication and mention of the grant of the patent: **17.04.2024**
(21) Application number: 17810346.1
(22) Date of filing: 07.06.2017
(51) Int. Cl.: A23L 35/00, A23K 10/20, A23L 33/10, A61K 35/64, A61P 37/04, A23L 5/20, A61K 35/63, A61P 3/02

(54) **PRODUCTION METHOD OF COMPOSITION USING INSECT AS A RAW MATERIAL**
HERSTELLUNGSVERFAHREN FÜR EINE ZUSAMMENSETZUNG UNTER VERWENDUNG VON INSEKTEN ALS ROHMATERIAL
MÉTHODE DE PRODUCTION D'UNE COMPOSITION EN UTILISANT DES INSECTES COMME MATIÈRE PREMIÈRE

(30) Priority: 07.06.2016 JP 2016113379
(43) Date of publication of application: 10.04.2019
(73) Proprietor: National University Corporation Ehime University, Matsuyama Ehime 790-08577 (JP)
(72) Inventor: MIURA, Takeshi, Matsuyama-shi Ehime 790-8566 (JP); MIURA, Chiemi, Matsuyama-shi Ehime 790-8566 (JP); OHTA, Takashi, Minamiuwa-gun Ehime 798-4292 (JP); HASHIZUME, Atsushi, Matsuyama-shi Ehime 790-8566 (JP)
(74) Representative: Greaves Brewster LLP
(86) International application number: PCT/JP2017/021144
(87) International publication number: WO 2017/213172

(56) References cited:
- WO-A1-2008/091137
- WO-A1-2014/017451
- JP-A- S4 848 276
- JP-A- S4 848 276
- JP-A- 2003 531 100
- JP-A- 2011 234 701
- KANKYO: "Anzen Data Sheet, Kagakuhin no Meisho: Hydroquinone", Anzen Data Sheet, 1 June 2016 (2016-06-01), pages 1-9, XP009517702, Seiri Bango 3767
- YOICHI ASO: "Oxidation of Catecholamines in Insects", Journal of Pesticide Science, vol. 19, no. 2, 1994, pages S61-S65, XP055562966,
- TAKESHI MIURA et al.: "Gyofun ni Kawaru Yogyo Shiryo Genryo Konchu Meal no Kanosei to Kinosei", Aqua Culture Business, vol. 52, no. 3, 1 March 2015 (2015-03-01), pages 35-39, XP9514530,

## Description

### TECHNICAL FIELD

The present invention relates to a production method of a composition for a food and/or a feed using an insect as a raw material, which comprises a step of removing fat-soluble components contained in the insect with a solvent.

### BACKGROUND ART

With the price of fish meal, an animal protein important as a food or a feed, surging worldwide, efforts to utilize insects that can be easily and efficiently produced from organic matters as a new alternative protein are under way. Specifically, a technique for processing fly larvae and/or pupae into a feed (Patent Document 1), a technique for generating fly larvae and/or pupae from organic waste (see, Patent Documents 2 to 4), and the like have been developed. Also, it has been found that polysaccharides having an immunostimulatory ability are contained in insects, and applications of the polysaccharides to aquaculture, livestock production and medical care are being investigated (see, Patent Document 6).

### CITATION LIST

### PATENT LITERATURE

[Patent Literature 1] International Publication WO 2011/007867
[Patent Literature 2] Japanese Laid-Open Patent Publication No. 2003-210071
[Patent Literature 3] Japanese Laid-Open Patent Publication No.3533466
[Patent Literature 4] Japanese Laid-Open Patent Publication No. H-10-215785
[Patent Literature 5] Japanese Laid-Open Patent Publication No. 3564457
[Patent Literature 6] International Publication WO 2014/017451

### SUMMARY OF INVENTION

### TECHNICAL PROBLEM

Up to now, the optimum content of insect larvae and/or pupae in feeds for aquaculture and/or livestock production containing larvae and/or pupae of insects such as flies and the like was about 0.5% by weight to about 25% by weight in terms of dry weight relative to the whole feed (see, Patent Document 1), and when giving a feed containing insect larvae and/or pupae beyond the above range, invitation of the growth retardation of the animals having fed the given feed was a problem. Although insects contain a polysaccharide having an immunostimulatory ability (see, Patent Document 6), if insects are provided as they are as a food or as a feed for aquaculture or livestock production, in a dried state, or in a state of being dried and powdered, the expected immunostimulatory effect could not be obtained in some cases.

### SOLUTION TO PROBLEM

The present inventors have analyzed insects that can be used as raw materials for foods and feeds in detail and resultantly revealed that insects contain components having a possibility of inducing immunosuppression, cytotoxicity and growth retardation in animals having fed the insects and further found a method for removing the components, leading to the present invention.

That is, the present invention is a production method of a composition for a food and/or a feed using an insect as a raw material, comprising a step of removing fat-soluble components including 1,2-benzenediol contained in the insect with a solvent, where the composition obtained by removing the fat-soluble components does not substantially contain 1,2-benzenediol,wherein the insect is exposed to said solvent for from 2 to 48 hours,wherein said solvent is one or more selected from the group consisting of hexane, acetone, benzene, toluene, ligroin, diethyl ether, petroleum ether, methyl acetate, ethyl acetate, cyclohexane, chloroform, ethanol, 1-propanol, 2-propanol, 1-butanol, 2-butanol, glycerol, propylene glycol, ethyl methyl ketone, 1,1,1,2-tetrafluoroethane, 1,1,2-trichloroethene, nitrous oxide, carbon dioxide, propane, butane and a mixture thereof,and wherein "does not substantially contain 1,2-benzenediol" means that the content of 1,2-benzenediol in the composition is lower than the content thereof at which 1,2-benzenediol acts on humans or animals having ingested the composition

In another production method of a composition of the present invention, 1,2-benzenediol is soluble in the solvent.

In another aspect of the present invention, the insect is one or more selected from the group consisting of insect larvae, pre-pupae, pupae, adults, eggs, dried products thereof, powders thereof and dry powders thereof. Further, the step of removing fat-soluble components contained in an insect with a solvent is a step of exposing the insect to the solvent to elute fat-soluble components contained in the insect into the solvent and further separating the solvent into which fat-soluble components of the insect have been eluted, from the insect.

The insect may belong to one or more members selected from the group consisting of family Muscidae, family Stratiomyidae, family Tephritidae, family Gryllidae, family Tenebrionidae, family Bombycidae and family Saturniidae.

The composition obtained by the step of removing fat-soluble components contained in an insect with a solvent is substantially free of 1,2-benzenediol. Furthermore, in another aspect of the present invention, the composition obtained by the step of removing fat-soluble components contained in an insect with a solvent has an immunostimulatory activity.

### ADVANTAGEOUS EFFECTS OF INVENTION

The composition for a food and/or a feed using an insect as a raw material produced according to the present invention is substantially free of 1,2-benzenediol and can be widely used as a food, or as a feed or feed raw material for aquaculture or livestock production. It is possible to provide a diet that does not cause an immunosuppressive action, toxicity or growth retardation in the fed animals or a food which is safe to humans who have ingested.

In addition, according to the production method of the present invention, a composition that can exert the effect of the polysaccharide can be obtained without purifying the polysaccharide contained in insects and having an immunostimulatory ability. Hence, the composition of the present invention can be used as a pharmaceutical, a health food or a nutritional supplement for humans, as a medicine for aquaculture and/or livestock production, or as a feed additive or a nutritional supplement.

### BRIEF DESCRIPTION OF DRAWINGS

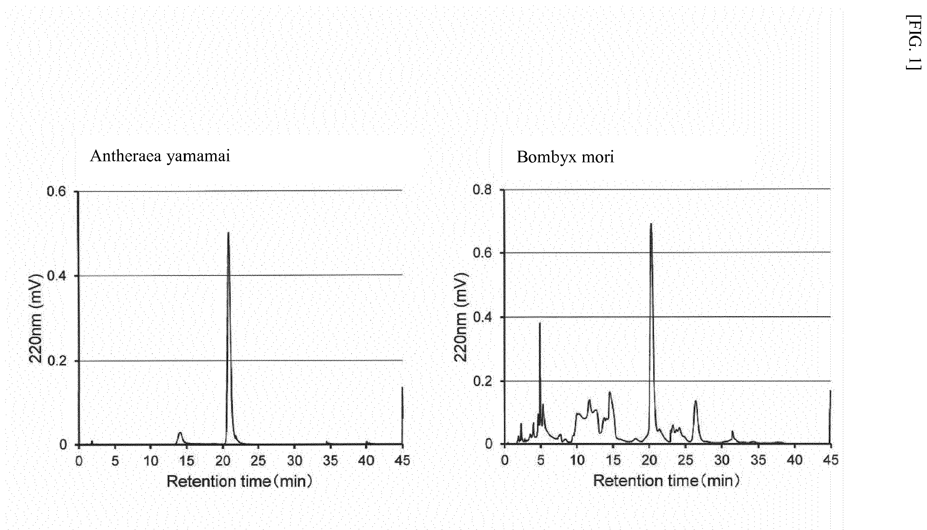

Fig. 1 shows an elution curve of high performance liquid chromatography of a fraction obtained from Antheraea yamamai and a fraction obtained from Bombyx mori.
Fig. 2 shows the results of structural analysis by a nuclear magnetic resonance method of a fraction obtained from Antheraea yamamai and a standard preparation of 1,2-benzenediol.
Fig. 3 shows an elution curve of high performance liquid chromatography of a fraction obtained from Antheraea yamamai and a standard preparation of 1,2-benzenediol.
Fig. 4 is a view showing an immunosuppressive action of 1,2-benzenediol.
Fig. 5 is a view showing cytotoxicity of 1,2-benzenediol.
Fig. 6 is a view showing the results of a breeding test using a feed containing the composition of the present invention.
Fig. 7 is a view showing the results of a breeding test using a feed containing the composition of the present invention.

### Modes for Carrying Out the Invention

The composition for a food and/or a feed using an insect as a raw material produced according to the present invention is a composition obtained by removing fat-soluble components contained in the insect with a solvent. The inventors have revealed that fat-soluble components of an insect include benzenediol and/or its derivative and that benzenediol and/or its derivative contained in an insect has a possibility of inducing immunosuppression, cytotoxicity or growth retardation in humans or animals who have ingested the insect, leading to the present invention.

Specifically, the insect in the present invention is one or more selected from the group consisting of insect larvae, pre-pupae, pupae, adults, eggs, dried products thereof, powders thereof and dry powders thereof. In order to enhance the efficiency of elution of fat-soluble components into a solvent, it is preferable that the insect is a dry product or a dry powder. The removal of fat-soluble components of an insect is carried out by exposing the insect to a solvent to elute fat-soluble components and separating the solvent containing the eluted fat-soluble components from the insect. The removal of fat-soluble components of an insect can be carried out once or plural times, but the number of times of the removal can be changed corresponding to the amount of fat-soluble components contained in the insect.

For exposing an insect to a solvent, specifically, an insect is immersed in a solvent, an insect is stirred in a solvent, or the like. Means for separating a solvent from an insect include a known solid-liquid separation method and the like. By separating the solvent containing the eluted fat-soluble components from the insect, it is possible to obtain a composition for a food and/or a feed composed of the insect from which fat-soluble components have been removed.

The time to expose an insect to a solvent is preferably 2 to 48 hours, and can be changed depending on the species and the state of the insect. For example, if the insect is in the form of a dry powder, the time of exposure to a solvent can be shortened so that fat-soluble components contained in the insect are eluted into the solvent efficiently.

At least 1,2-benzenediol is soluble in the solvent used for removing fat-soluble components from an insect. Specifically, the solvent can be an organic solvent. The composition for a food and/or a feed obtained by treating as described above with a solvent in which 1,2-benzenediol is soluble is substantially free of 1,2-benzenediol .

The solvent used for removing fat-soluble components from an insect includes, more specifically, one or more selected from the group consisting of hexane, acetone, benzene, toluene, ligroin, diethyl ether, petroleum ether, methyl acetate, ethyl acetate, dichloromethane, cyclohexane, chloroform, methanol, ethanol, 1-propanol, 2-propanol, 1-butanol, 2-butanol, glycerin, propylene glycol, ethyl methyl ketone, 1,1,1,2-tetrafluoroethane, 1,1,2-trichloroethene, nitrous oxide, carbon dioxide, methane, ethane, ethylene, propane, pentane, butane, and a mixture thereof. The solvent such as carbon dioxide, methane, ethane, ethylene, propane, butane, methanol and ethanol can be used in the state of supercritical fluid or subcritical fluid.

The composition for a food and/or a feed using an insect as a raw material produced by the production method comprising a step of removing fat-soluble components with a solvent is superior in safety to humans or animals who ingest the composition, as compared with a composition produced by a production method not comprising a step of removing fat-soluble components.

The diet containing the composition produced by the production method of the present invention can obtain effects such as an improvement in the feed intake, an improvement in the growth amount, an improvement in the feed efficiency and the like of livestock animals or aquatic animals, as compared with a feed containing a composition produced by a production method not including a step of removing fat-soluble components. In particular, the composition produced by the production method of the present invention can be used as a substitute for fish meal produced from natural fish such as anchovy and the like and the processing residues thereof. By using the composition of the present invention, it is possible to provide a feed giving excellent growth of animals having fed the composition without using fish meal.

The solvent used for removing fat-soluble components from an insect is preferably one having a property that components useful as a food and/or a feed contained in the insect, such as proteins and polysaccharides and the like, are difficult to elute. In particular, insects contain a polysaccharide having an immunostimulatory activity (see, Patent Document 6), but the immunostimulatory activity possessed by the polysaccharide is suppressed by components such as benzenediol and/or its derivative contained in the insect in some cases. The composition for a food and/or a feed using an insect as a raw material produced by the production method comprising a step of removing fat-soluble components with a solvent can exert a higher immunostimulatory activity, as compared with a composition produced by a production method not including a step of removing fat-soluble components.

Hence, as the solvent used for removing fat-soluble components from an insect, a solvent in which the polysaccharide is insoluble or difficult to elute described in Patent Document 6 is preferable. Specific examples thereof include low polar solvents such as hexane, ligroin, diethyl ether, petroleum ether and the like, and ethanol, mixed solvents thereof, and the like.

The composition produced by the production method of the present invention can be a composition substantially not containing benzenediol and/or its derivative. "Substantially not containing benzenediol and/or its derivative" means that the composition contains benzenediol and/or its derivative only to the extent that the content of benzenediol and/or its derivative in the composition is lower than the content thereof at which benzenediol and/or its derivative acts on humans or animals having ingested the composition. More specifically, it means that benzenediol and/or its derivative is contained only in an amount that does not exert an effect such as immunosuppression, toxicity, growth retardation and the like in a human body ingesting as a food a composition using an insect as a raw material or in an animal body ingesting the composition as a feed.

The amount to such an extent that it does not exert any action such as immunosuppression, toxicity, growth retardation and the like in the body of an animal that eats an insect as a feed or a feed raw material specifically denotes an amount to such an extent that when a solvent such as acetone and the like in which benzenediol and/or its derivative is soluble is added to a measuring object and the mixture is stirred and centrifugally separated to obtain a supernatant which is then quantified using a gas chromatography mass spectrometer (GC/MS), the detection of benzenediol and/or its derivative is difficult, more specifically denotes an amount lower than 1 µg/g. Accordingly, one embodiment is a composition composed of insect larvae, pre-pupae, pupae, adults or eggs substantially not containing benzenediol and/or its derivative, and yet another embodiment thereof is a composition composed of insect larvae, pre-pupae, pupae, adults or eggs wherein the content of benzenediol and/or its derivative is less than 1 µg/g.

Benzenediol is an organic compound in which two hydroxy groups are substituted on the benzene ring, and as structural isomers, 1,2-benzenediol (also called "catechol"), 1,3- benzenediol (also called "resorcinol") and 1,4- benzenediol (also called "hydroxyquinone") are present. Benzenediol has strong reducing power and has been reported to have strong toxicity to animals and plants.

Examples of derivatives of benzenediol include those containing a substituent on a part of benzenediol. More specifically, examples of the substituent include an alkyl group, an alkenyl group, an allyl group, a vinyl group, an ether group, an ester group, a phenyl group, an aryl group, a benzyl group, a benzoyl group, a naphthyl group, a halogen group, a hydroxy group, an acyl group, an acetyl group, an aldehyde group, an amino group, an alkoxy group, a nitro group, an azi group, a cyano group, an azide group, a thiol group, a sulfo group, a chloro group and the like.

The insects which are the raw materials of the composition of the present invention are generic names of organisms belonging to Arthropoda in the animal kingdom and classified as Insecta. Insecta is classified into Apterygota and Pterygota. Apterygota is classified into Collembola, Protura, Thysanura and the like. Further, Pterygota is classified into Ephemeroptera, Plecoptera, Odonata, Embioptera, Orthoptera, Dermaptera, Isoptera (order termite), Embioptera (order web spinner), Psocoptera, Mallophaga, Anoplura, Thysanoptera, Hemiptera, Hymenoptera, Strepsiptera, Coleoptera, Neuroptera, Mecoptera, Trichoptera, Lepidoptera, Diptera, Aphaniptera and the like.

It is believed that benzenediol and/or its derivatives are generally contained in insects, such as one of benzenediol and/or its derivatives, catecholamine, is known to be involved in growth of insect larvae. On the other hand, when insects are used as a food or a feed, it is considered that insects that can be easily and efficiently reared or insect species with established artificial rearing methods are suitable. Specifically, the order Diptera includes Musca domestica belonging to the family Muscidae; Sarcophaga peregrina belonging to the family Sarcophagidae; Tetradacustsuneonis, Strumeta dorsalis and Bactrocera cucurbitae/Zeugodacus cucurbitae belonging to the family Tephritidae; Hermetia illucens and Ptecticustenebrifer belonging to the family Stratiomyiidae; and the like.

In addition, the order Lepidoptera includes Bombyx mori belonging to the family Bombycidae; and Samia Cynthia pryeri, Antheraeapernyi, Antheraea yamamai (also called giant silkworm moth, or wild silkworm), Gonimbrasia belina and the like belonging to the family Saturniidae. The order Coleoptera includes Tenebrio obscurus, Tenebriomolitor, Zophobas atratus Fabricius and the like, so called meal worms, belonging to the superfamily Tenebrionoidea and the family Tenebrionidae, and the order Orthoptera includes Gryllus bimaculatus, Acheta domesticus and the like belonging to the family Grylloidea. However, the present invention is not limited to these specifically exemplified insect species.

Insect larvae, pre-pupae, pupae, adults or eggs can be used as a raw material for the composition of the present invention, but when the composition of the present invention is used as a feed or a feed raw material, insect larvae, pre-pupae or pupa are preferably used.

The composition of the present invention can be supplied to an aquaculture animal and/or a livestock animal as a feed for aquaculture and/or animal husbandry, but it may also be provided as a mixed diet containing the composition as one of raw materials. Specifically, the composition of the present invention can be used as a raw material for replacing fish meal, which is a raw material for supplying animal proteins.

Furthermore, since the composition obtainable by the present invention exerts the effect of the polysaccharide having an immunostimulatory activity contained in insects, it can be used not only as a medicine for aquaculture animals and/or livestock animals or as a feed additive or a nutritional supplement, but also can be used as a medicine for humans or as a health food or a nutritional supplement.

### EXAMPLES

Hereinafter, the present invention will be described in more detail with reference to examples below, but the present invention is not limited to the following examples.

### 1. Identification of substance with immunosuppressive action contained in insect

A substance contained in Antheraea yamamai pupae and Bombyx mori pupae and having an immunosuppressive action on humans or animals having ate the insects was identified. Distilled water was added to a dry powder of Antheraea yamamai pupae and a dried powder of Bombyx mori pupae and the mixture was stirred and centrifugally separated to obtain the supernatant. Ethyl acetate was added to the resultant supernatant to cause liquid-liquid partition, and the resultant ethyl acetate layer was evaporated to dryness by distilling off the solvent with a rotary evaporator. Distilled water was added to the residue, which was added to a C18 solid phase column, and the flow-through fraction was recovered. The resultant flow-through fraction was eluted with high performance liquid chromatography (Develosil C30 column), and the immunosuppressive action of each eluted fraction obtained was measured. For measurement of the immunosuppressive action, LPS was added to a culture solution of a mouse macrophage cell line (RAW 264 cells) so as to be 100 ng/mL, each eluted fraction was further added, and the nitrogen monoxide production inhibition rate by each eluted fraction was determined. Inhibition of nitrogen monoxide production was confirmed in the eluted fraction containing the peak at 220 nm (mV) of the chromatogram shown in Fig. Thus, it was shown that a substance having an immunosuppressive action is present in the eluted fraction.

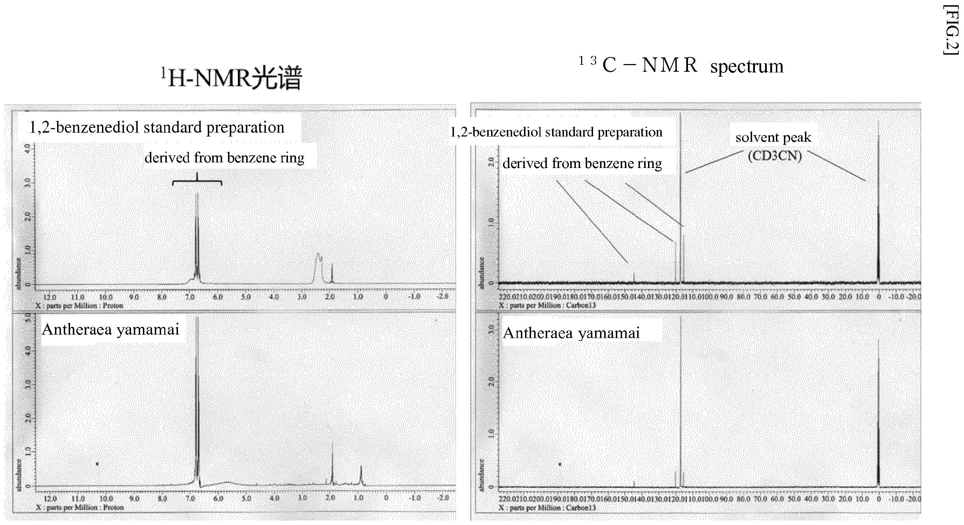

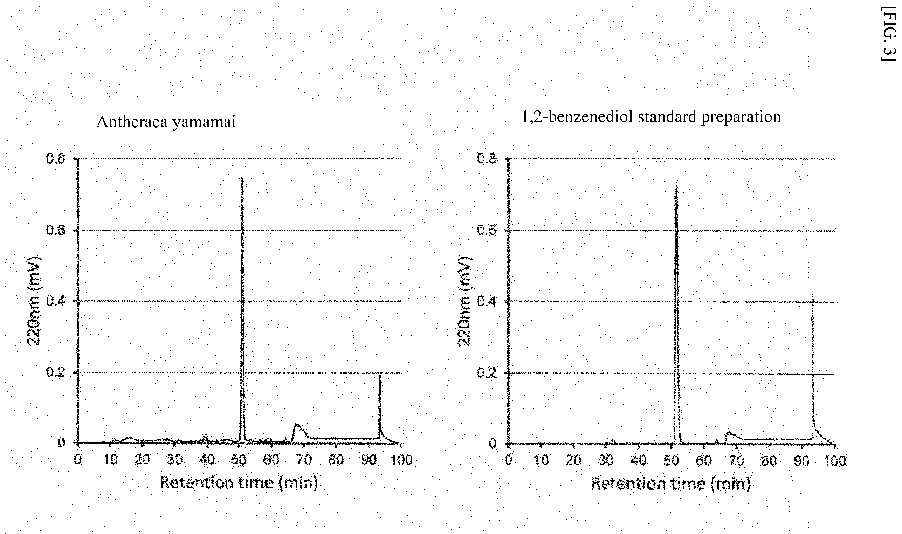

Further, structural analysis of the substance contained in the eluted fraction was carried out by a nuclear magnetic resonance method. As shown in FIG. 2, the NMR spectrum of the sample obtained from the fraction derived from Antheraea yamamai pupae and the NMR spectrum of 1,2-benzenediol matched, in the 1H-NMR spectrum and the 13C-NMR spectrum. Further, when a standard preparation of 1,2-benzenediol was eluted by high performance liquid chromatography (COSMOSIL 5C18ARII column), the peak at 220 nm (mV) indicated by the eluted fraction obtained from Antheraea yamamai pupae and the peak of a standard preparation of 1,2-benzenediol agreed with each other, as shown in FIG. 3. Thus, it was clarified that the substance having an immunosuppressive action contained in Antheraea yamamai pupae and Bombyx mori pupae is 1,2-benzenediol and/or its derivative.

### 2. Evaluation of immunosuppressive action of benzenediol

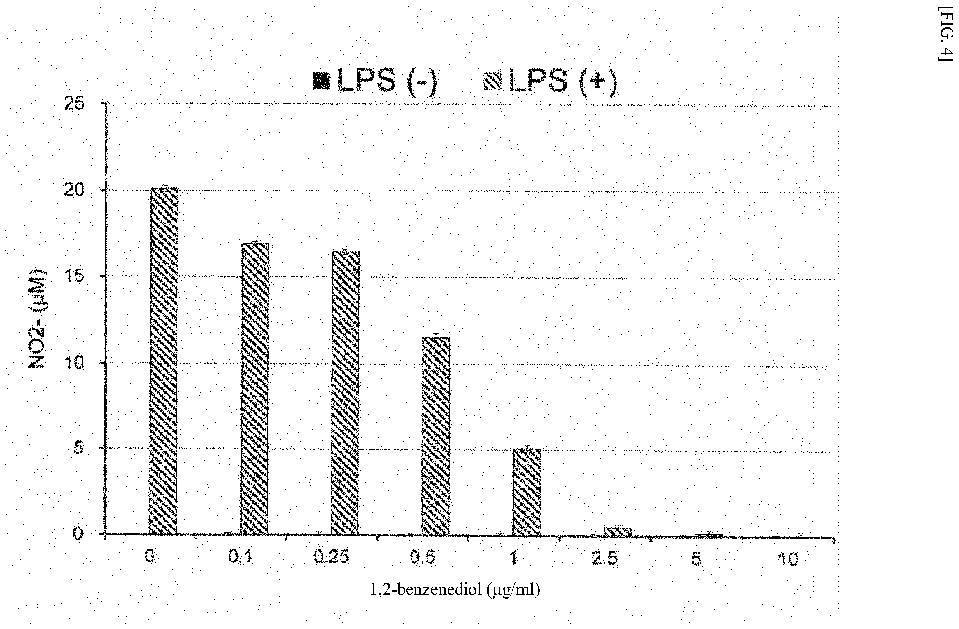

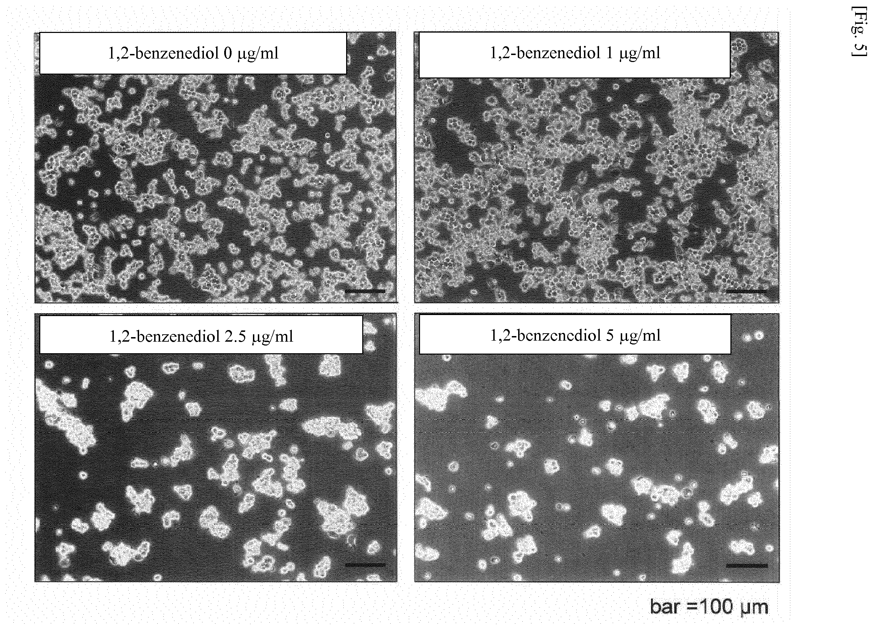

Whether 1,2-benzenediol identified from Antheraea yamamai pupae and Bombyx mori pupae has an immunosuppressive action or not was evaluated by the production of nitrogen monoxide from a mouse macrophage cell line (RAW264 cells) as an indicator. When lipopolysaccharide (LPS) was added to the culture solution to 100 ng/mL and 1,2-benzenediol was further added so as to become 0 µg/mL to 10 µg/mL, inhibition of nitrogen monoxide production was observed depending on the concentration of 1,2-benzenediol, as shown in FIG. 4. In addition, as shown in FIG. 5, growth inhibition of a mouse macrophage cell line was confirmed in the presence of 1 µg/mL or more of 1,2-benzenediol. It was therefore suggested that benzenediol and/or its derivatives have not only an immunosuppressive action but also strong cytotoxicity.

### 3. Detection of benzenediol and/or its derivative in composition wherein fat-soluble components contained in insect have been removed

The content of 1,2-benzenediol contained in insects of various species was measured and the change of the content by removal of fat-soluble components contained in insects was verified. A supernatant obtained by adding 10-fold amount of 80% acetone to the object to be measured and stirring and centrifuging was used to quantify 1,2-benzenediol using a gas chromatography mass spectrometer (GC/MS) and the amount of 1,2-benzenediol contained in the object to be measured was clarified.

Fat-soluble components contained in a dry powder of insect larvae, pre-pupae or pupae were removed using various solvents. In "hexane-ethanol mixed treatment", a mixture obtained by mixing hexane:ethanol = 9:1 was added in an amount about 4 times the dry powder of the insect and mixed while stirring at room temperature for 12 hours to 24 hours, then, the solvent was removed by centrifugal separation or the like, and dried by a dryer at 40°C to 80°C for 12 hours to 24 hours.

Further, in "hexane treatment", hexane was added in an amount about 4 times the dry insects, mixed while stirring at room temperature for 12 hours to 24 hours, then, the solvent was removed by centrifugal separation or the like, and dried by a dryer at 40°C to 80°C for 12 hours to 24 hours. In "ethanol treatment", ethanol was added in an amount about 4 times the dry insects, mixed while stirring at room temperature for 12 hours to 24 hours, then, the solvent was removed by centrifugal separation or the like, and dried by a dryer at 40°C to 80°C for 12 hours to 24 hours.

In "acetone treatment", acetone was added in an amount about 4 times the dry insects, mixed while stirring at room temperature for 12 hours to 24 hours, then, the solvent was removed by centrifugal separation or the like, and dried by a dryer at 40°C to 80°C for 12 hours to 24 hours. In "ethyl acetate process", ethyl acetate was added in an amount about 4 times the dry insects, mixed while stirring at room temperature for 12 hours to 24 hours, the solvent was removed by centrifugal separation or the like, and dried by a dryer at 40°C to 80°C for 12 hours to 24 hours.

In "ligroin treatment", ligroin was added in an amount about 4 times the dry insects, mixed while stirring at room temperature for 12 hours to 24 hours, then, the solvent was removed by centrifugal separation or the like, and dried by a dryer at 40°C to 80°C for 12 hours to 24 hours. In "petroleum ether treatment", petroleum ether was added in an amount about 4 times the dry insects, mixed while stirring at room temperature for 12 hours to 24 hours, the solvent was removed by centrifugal separation or the like, and dried by a dryer at 40°C to 80°C for 12 hours to 24 hours. In "diethyl ether treatment", diethyl ether was added in an amount about 4 times the dry insects, mixed while stirring at room temperature for 12 hours to 24 hours, the solvent was removed by centrifugal separation or the like, and dried by a dryer at 40°C to 80°C for 12 hours to 24 hours.

Tables 1 to 5 show the results of removal of fat-soluble components by the above-described treatments and the quantitative results by a gas chromatography mass spectrometer. The conditions under which the fat-soluble components were removed are indicated by "o" in the table. From 9.3 µg/g to 200 µg/g of 1,2-benzenediol was detected from insects before removal of fat-soluble components, but it was found that compositions composed of larvae, pre-pupae or pupae of insects from which fat-soluble components had been removed by the treatment with each solvent and containing substantially no benzenediol and/or its derivative were obtained. In addition, the content of 1,2-benzenediol of the composition containing insect larvae, pre-pupae or pupae as a raw material in this example was considered to be less than 1 µg/g since the minimum detectable amount of 1,2-benzenediol was 1 µg/g in the quantitative method adopted in this example.

**[Table 1]**

| insect | solvent | removal of fat-soluble components | 1,2-benzenediol content (µg/g) |
|---|---|---|---|
| Musca domestica larvae (Diptera, Muscifae) | no treatment | - | 27.1 |
| | hexane-ethanol mixture | ○ | no-detection |
| | hexane | ○ | no-detection |
| | ethanol | ○ | no-detection |
| | acetone | ○ | no-detection |
| | ethyl acetate | ○ | no-detection |
| | ligroin | ○ | no-detection |
| | petroleum ether | ○ | no-detection |
| Musca domestica Pupa (Diptera, Muscidae) | no treatment | - | 21.7 |
| | hexane-ethanol mixture | ○ | no-detection |
| | hexane | ○ | no-detection |
| | ethanol | ○ | no-detection |
| | acetone | ○ | no-detection |
| | ethvl acetate | ○ | no-detection |
| | ligroin | ○ | no-detection |
| | petroleum ether | ○ | no-detection |

| | | | |
|---|---|---|---|
| minimum detectable amount: 1 µg/g | | | |

**[Table 2]**

| insect | solvent | removal of fat-soluble components | 1,2-benzenediol content (µg/g) |
|---|---|---|---|
| Hermetia illucens larva (Diptera, Stratiomyiidae) | no treatment | - | 9.3 |
| | hexane-ethanol mixture | ○ | no-detection |
| | hexane | ○ | no-detection |
| | ethanol | ○ | no-detection |
| | acetone | ○ | no-detection |
| | ethyl acetate | ○ | no-detection |
| | ligroin | ○ | no-detection |
| | petroleum ether | ○ | no-detection |
| Hermetia illucens prepupa (Diptera, Stratiomyiidae) | hexane-ethanol mixture | ○ | no-detection |
| | hexane | ○ | no-detection |
| | ethanol | ○ | no-detection |
| | acetone | ○ | no-detection |
| | ethyl acetate | ○ | no-detection |
| | ligroin | ○ | no-detection |
| | petroleum ether | ○ | no-detection |

| | | | |
|---|---|---|---|
| minimum detectable amount: 1 µg/g | | | |

**[Table 3]**

| insect | solvent | removal of fat-soluble components | 1,2-benzenediol content (µg/g) |
|---|---|---|---|
| Bombyx mori pupa (Lepidoptera, Bombycidae) | no treatment | - | 135.3 |
| | hexane-ethanol mixture | ○ | no-detection |
| | hexane | ○ | no-detection |
| | ethanol | ○ | no-detection |
| | acetone | ○ | no-detection |
| | ethyl acetate | ○ | no-detection |
| | ligroin | ○ | no-detection |
| | petroleum ether | ○ | no-detection |
| | diethyl ether | ○ | no-detection |

**[Table 4]**

| insect | solvent | removal of fat-soluble components | 1,2-benzenediol content (µg/g) |
|---|---|---|---|
| Antheraea yamamai pupa (Lepidoptera, Saturniidae) | no treatment | - | 200.0 |
| | hexane-ethanol mixture | ○ | no-detection |
| | hexane | ○ | no-detection |
| | ethanol | ○ | no-detection |
| | acetone | ○ | no-detection |
| | ethyl acetate | ○ | no-detection |
| | ligroin | ○ | no-detection |
| | petroleum ether | ○ | no-detection |

**[Table 5]**

| insect | solvent | removal of fat-soluble components | 1,2-benzenediol content (µg/g) |
|---|---|---|---|
| Mealworm larva (Coleoptera, Tenebrionidae) | no treatment | - | 16.0 |
| | hexane-ethanol mixture | ○ | no-detection |
| | hexane | ○ | no-detection |
| | ethanol | ○ | no-detection |
| | acetone | ○ | no-detection |
| | ethyl acetate | ○ | no-detection |
| | ligroin | ○ | no-detection |
| | petroleum ether | ○ | no-detection |

In addition, Orthoptera Gryllus bimaculatus larvae and Acheta domesticus larvae were treated with the mixed solvent of hexane and ethanol in the same manner as described above, as a result, 1,2-benzenediol was not detected.

### 4. Immunostimulatory test of composition wherein fat-soluble components contained in insect have been removed

The immunostimulatory ability possessed by the composition wherein fat-soluble components contained in insects had been removed was compared with those contained in insects not subjected to the step of removing fat-soluble components. To 1 g of the object to be measured, 10 mL of a buffer solution (20 mM Tris-HCl pH 8.0,150 mM NaCl) was added and the supernatant vigorously shaken at room temperature for 1 day to 2 days was filtrated through a filter, and diluted with a cell culture solution. The culture solution containing the diluted sample was added to a mouse macrophage cell line (RAW 264 cells) and cultured for 24 hours, and the culture supernatant was collected and the production amount of nitrogen monoxide was measured.

The results are shown in the following Tables 6 to 8. It was revealed that in the composition wherein fat-soluble components contained in insects had been removed, the amount of nitrogen monoxide production in a mouse macrophage cell line was higher than that of the untreated insect. Therefore, it was suggested that a composition with a high immunostimulatory activity was obtained by removal of fat-soluble components from insects.

**[Table 6]**

| insect | removal step | dilution ratio | NO output (µM) |
|---|---|---|---|
| Bombyx mori pupa | no treatment | 1/10 | 16.4 |
| | ligroin | 1/10 | 29.2 |

**[Table7]**

| insect | treatment method | dilution ratio | NO output (µM) |
|---|---|---|---|
| Bombyx mori pupa | no treatment | 1/200 | 23.1 |
| | hexane | 1/200 | 27.3 |

**[Table 8]**

| insect | treatment method | dilution ratio | NO output (µM) |
|---|---|---|---|
| Antheraea yamamai pupa | no treatment | 1/50 | 20.4 |
| | hexane | 1/50 | 21.3 |

### 5. Feeding test with diets containing composition using insect as raw material produced by production method including step of removing fat-soluble components contained in insect with solvent

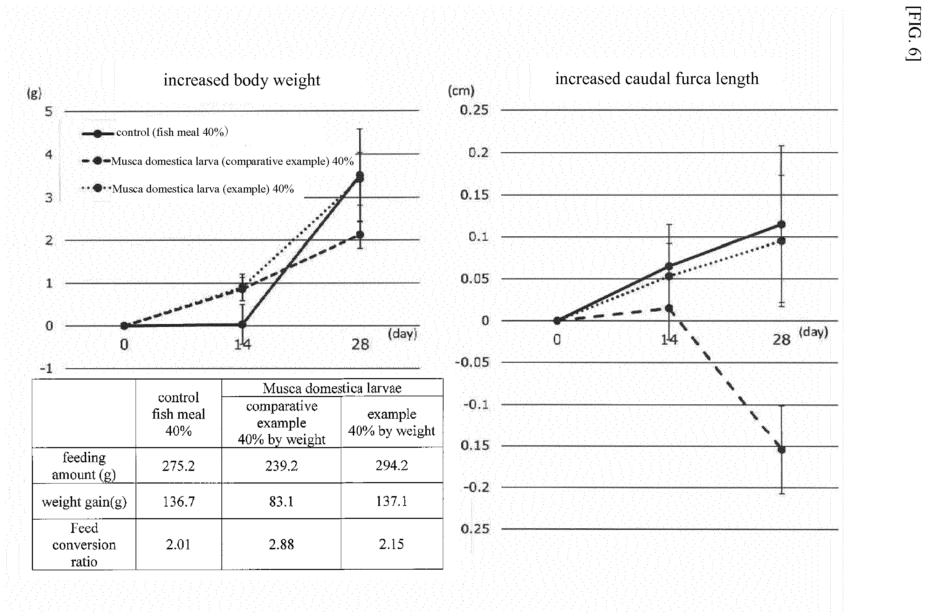

A diet composed of a composition consisting of a dried powder of Musca domestica larvae (hereinafter referred to as "Comparative Example") as a feed raw material and a feed composed of a composition obtained by removing fat-soluble components from dry Musca domestica larvae with a solvent prepared by mixing hexane and ethanol at a ratio of 9:1 (hereinafter referred to as "Example") as a feed raw material were compared in a growth test. As the test fish, Pagrus major (madai) juvenile fish was used, and a diet containing fish meal made from anchovy in an amount of 40% by weight in terms of dry substance was used as a control, and fish meals were completely substituted by them in Example and Comparative Example, and a feeding test was carried out for 28 days. The results are shown in FIG. 6. For Pargus major fed with the diet containing dry 40% by weight of the composition of Musca domestica larvae of Example, approximately the same growth as in the control group was obtained, however, for Pagrus major fed with the diet containing dry 40% by weight of the composition of Musca domestica larvae of Comparative Example, the increased body weight and the increased fork length were significantly lowered as compared with the control group. Therefore, it was found that the diet using Musca domestica larvae from which fat-soluble components had been removed with a solvent, as a feed raw material, was effective.

Subsequently, a diet composed of a composition obtained by removing fat-soluble components from dried mealworm larvae with a solvent prepared by mixing hexane and ethanol at a ratio of 9:1 (hereinafter referred to as "Example") as a feed raw material was subjected to a growth comparison test. The compositions of the test diets are shown in Table 9. In the diet of test group 3, the fat-soluble components removed in producing Example were added as an oil component so that the oil contents of feeds used in the areas were equal.

**[Table 9]**

| | examples | fish meal | Oil |
|---|---|---|---|
| test group 1 | 65% by weight | 0% by weight | fish oil |
| test group 2 | 40% by weight | 25% by weight | fish oil |
| test group 3 | 65% by weight | 0% by weight | fat-soluble component obtained from larva of mealworm |
| control group | 0% by weight | 65% by weight | fish oil |

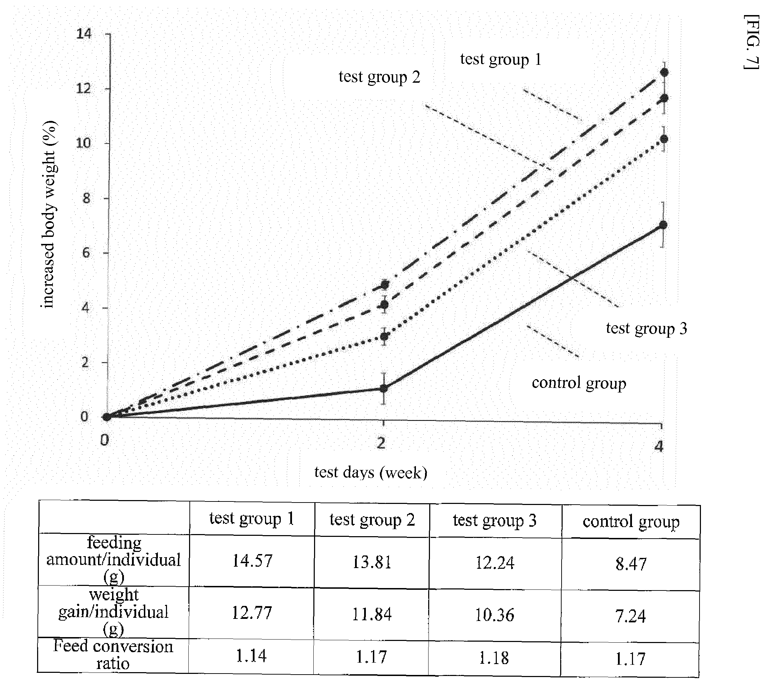

As the test fish, Pagrus major juvenile fish was used, and a diet containing fish meal made of anchovy in an amount of 65% by weight in terms of dry substance was used as a control, and fish meals were completely substituted by them in Example and Comparative Example, and a feeding test was carried out for 28 days by satiety feeding. The results are shown in FIG. 7. For the diet containing the composition of Example, the feeding amount was larger and growth was superior to compared with the diet of the control group. Particularly, in the test group 1 in which the fish meals were substituted completely with Example, the maximum feeding amount and increased weight gain were observed. In contrast, in the feed in which fat-soluble components removed by a solvent had been added, the feeding amount and the increased weight were apparently inferior to those with the diets containing fish oil added, hence, it was considered that a substance inducing growth retardation was contained in the fat-soluble components. Thus, it was found that the feed using mealworm larvae from which fat-soluble components had been removed with a solvent, as a feed raw material, was effective.

## Claims

1. A production method of a composition for a food and/or a feed using an insect as a raw material, comprising a step of removing fat-soluble components including 1,2-benzenediol contained in the insect with a solvent, where the composition obtained by removing the fat-soluble components does not substantially contain 1,2-benzenediol,
wherein the insect is exposed to said solvent for from 2 to 48 hours,
wherein said solvent is one or more selected from the group consisting of hexane, acetone, benzene, toluene, ligroin, diethyl ether, petroleum ether, methyl acetate, ethyl acetate, cyclohexane, chloroform, ethanol, 1-propanol, 2-propanol, 1-butanol, 2-butanol, glycerol, propylene glycol, ethyl methyl ketone, 1,1,1,2-tetrafluoroethane, 1,1,2-trichloroethene, nitrous oxide, carbon dioxide, propane, butane and a mixture thereof,
and wherein "does not substantially contain 1,2-benzenediol" means that the content of 1,2-benzenediol in the composition is lower than the content thereof at which 1,2-benzenediol acts on humans or animals having ingested the composition.

2. The production method of the composition according to Claim 1, wherein 1,2-benzenediol is soluble in said solvent.

3. The production method of the composition according to claim 1 or claim 2, wherein said insect is one or more selected from the group consisting of insect larvae, pre-pupae, pupae, adults, eggs, dried products thereof, powders thereof and dry powders thereof, and said step is a step of exposing the insect to the solvent to elute fat-soluble components contained in the insect into the solvent and further separating the solvent into which the fat-soluble components of the insect have been eluted, from the insect.

4. The production method of the composition according to any preceding claim, wherein said composition obtained by removing the fat-soluble components has an immunostimulatory activity.

5. The production method of the composition according to any preceding claim, wherein said insect belongs to Lepidoptera.

## Patentansprüche

1. Herstellungsverfahren einer Zusammensetzung für ein Nahrungsmittel und/oder ein Futtermittel unter Verwendung eines Insekts als ein Rohmaterial, umfassend einen Schritt eines Entfernens fettlöslicher Komponenten, einschließlich 1,2-Benzoldiol, die in dem Insekt enthalten sind, mit einem Lösungsmittel, wobei die Zusammensetzung, die durch das Entfernen der fettlöslichen Komponenten erhalten wird, im Wesentlichen kein 1,2-Benzoldiol enthält,
wobei das Insekt dem Lösungsmittel von 2 bis 48 Stunden lang ausgesetzt wird,
wobei das Lösungsmittel eines oder mehrere ist, die aus der Gruppe ausgewählt sind, die aus Hexan, Aceton, Benzol, Toluol, Ligroin, Diethylether, Petrolether, Methylacetat, Ethylacetat, Cyclohexan, Chloroform, Ethanol, 1-Propanol, 2-Propanol, 1-Butanol, 2-Butanol, Glycerin, Propylenglykol, Ethylmethylketon, 1,1,1,2-Tetrafluorethan, 1,1,2-Trichlorethen, Lachgas, Kohlendioxid, Propan, Butan und einer Mischung davon besteht,
und wobei "enthält im Wesentlichen kein 1,2-Benzoldiol" bedeutet, dass der Gehalt an 1,2-Benzoldiol in der Zusammensetzung niedriger ist als der Gehalt davon, bei dem 1,2-Benzoldiol auf Menschen oder Tiere wirkt, die die Zusammensetzung aufgenommen haben.

2. Herstellungsverfahren der Zusammensetzung nach Anspruch 1, wobei 1,2-Benzoldiol in dem Lösungsmittel löslich ist.

3. Herstellungsverfahren der Zusammensetzung nach Anspruch 1 oder Anspruch 2, wobei das Insekt eines oder mehrere ist, die aus der Gruppe ausgewählt sind, die aus Insektenlarven, Vorpuppen, Puppen, adulten Insekten, Eiern, getrockneten Produkten davon, Pulvern davon und Trockenpulvern davon besteht, und wobei der Schritt ein Schritt eines Aussetzens des Insekts gegenüber dem Lösungsmittel, um in dem Insekt enthaltene fettlösliche Komponenten in das Lösungsmittel herauszulösen, und ferner eines Trennens das Lösungsmittels, in das die fettlöslichen Komponenten des Insekts herausgelöst wurden, von dem Insekt ist.

4. Herstellungsverfahren der Zusammensetzung nach einem vorhergehenden Anspruch, wobei die durch Entfernen der fettlöslichen Komponenten erhaltene Zusammensetzung eine immunstimulierende Aktivität aufweist.

5. Herstellungsverfahren der Zusammensetzung nach einem vorhergehenden Anspruch, wobei das Insekt zu den Lepidoptera gehört.

## Revendications

1. Méthode de production d'une composition pour un aliment et/ou un aliment pour animaux en utilisant un insecte comme matière première, comprenant une étape d'élimination des composants liposolubles comprenant le 1,2-benzènediol contenu dans l'insecte avec un solvant, où la composition obtenue par l'élimination des composants liposolubles ne contient sensiblement pas de 1,2-benzènediol,
dans laquelle l'insecte est exposé audit solvant pendant 2 à 48 heures,
dans laquelle ledit solvant est un ou plusieurs choisis dans le groupe constitué de l'hexane, acétone, benzène, toluène, ligroïne, éther diéthylique, éther de pétrole, acétate de méthyle, acétate d'éthyle, cyclohexane, chloroforme, éthanol, 1-propanol, 2-propanol, 1-butanol, 2-butanol, glycérol, propylène glycol, éthylméthylcétone, 1,1,1,2-tétrafluoroéthane, 1,1,2-trichloroéthène, oxyde nitreux, dioxyde de carbone, propane, butane et un mélange de ceux-ci,
et dans laquelle « ne contient sensiblement pas de 1,2-benzènediol » signifie que la teneur en 1,2-benzènediol dans la composition est inférieure à la teneur en celui-ci à laquelle le 1,2-benzènediol agit sur les humains ou les animaux ayant ingéré la composition.

2. Méthode de production de la composition selon la revendication 1, dans laquelle le 1,2-benzènediol est soluble dans ledit solvant.

3. Méthode de production de la composition selon la revendication 1 ou la revendication 2, dans laquelle ledit insecte est un ou plusieurs choisis dans le groupe constitué des larves d'insectes, pré-pupes, pupes, adultes, oeufs, produits séchés de ceux-ci, poudres de ceux-ci et poudres sèches de ceux-ci, et ladite étape est une étape d'exposition de l'insecte au solvant pour éluer les composants liposolubles contenus dans l'insecte dans le solvant et en outre de séparation du solvant dans lequel les composants liposolubles de l'insecte ont été élués, de l'insecte.

4. Méthode de production de la composition selon l'une quelconque des revendications précédentes, dans laquelle ladite composition obtenue en éliminant les composants liposolubles comporte une activité immunostimulatrice.

5. Méthode de production de la composition selon l'une quelconque des revendications précédentes, dans laquelle ledit insecte appartient aux Lépidoptères.
